(19) **Europäisches Patentamt / European Patent Office / Office européen des brevets**

(11) **EP 2 394 695 B1**

(12) **FASCICULE DE BREVET EUROPEEN**

(45) Date de publication et mention
de la délivrance du brevet:
**26.09.2012 Bulletin 2012/39**

(51) Int Cl.:
***A61N 1/05*** *(2006.01)*     ***A61N 1/375*** *(2006.01)*

(21) Numéro de dépôt: **11157822.5**

(22) Date de dépôt: **11.03.2011**

(54) **Capsule intracardiaque autonome et son accessoire d'implantation**

Autonome intrakardiale Kapsel, und entsprechendes Implantationszubehör

Standalone intracardiac capsule and implantation accessory

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.06.2010 FR 1054699**

(43) Date de publication de la demande:
**14.12.2011 Bulletin 2011/50**

(73) Titulaire: **Sorin CRM SAS**
**92143 Clamart Cedex (FR)**

(72) Inventeur: **Ollivier, Jean-François**
**91190 Villiers Le Bacle (FR)**

(74) Mandataire: **Dupuis-Latour, Dominique**
**Bardehle Pagenberg**
**10, boulevard Haussmann**
**75009 Paris (FR)**

(56) Documents cités:
**WO-A1-2007/067231    WO-A1-2009/039400**
**US-A1- 2009 082 827    US-A1- 2009 204 170**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne les "dispositifs médicaux implantables actifs" tels que définis par la directive 90/385/CEE du 20 juin 1990 du Conseil des communautés européennes; plus les implants permettant de surveiller en continu le rythme cardiaque et délivrer si nécessaire au coeur des impulsions électriques de stimulation, de de cardioversion et/ou de défibrillation en cas de trouble du rythme détecté par le dispositif,

Elle concerne plus particuliérement ceux de ces dispositifs qui mettent en oeuvre des capsules autonomes implantées dans une cavité cardiaque, ventricule ou oreillette, et qui sont dépourvues de toute liaison physique à un dispositif principal implanté (tel qu'un boîtier de générateur d'impulsions de stimulation) ou non implanté (périphérique externe tel que programmateur ou dispositif de monitoring pour le suivi à distance du patient).

Ces capsules autonomes sont dénommées pour cette raison "capsules *leadless*", pour les distinguer des électrodes ou des capteurs disposés à l'extrémité distale d'une sonde (*lead*), cette sonde étant parcourue sur toute sa longueur par un ou plusieurs conducteurs reliant par voie galvanique l'électrode ou le capteur à un générateur connecté à l'extrémité opposée, proximale, de la sonde.

De telles capsules *leadless* sont par exemple décrites dans les US 2007/0088397 A1 et WO 2007/047681 A2 (Nanostim. Inc.) ou encore dans le US 2008/0136004 A1 (EBR Systems, Inc.)

La fixation à la paroi cardiaque de ces capsules se fait généralement au moyen d'une vis d'ancrage hélicoïdale saillante, prolongeant axialement le corps cylindrique de la capsule et destinée à pénétrer dans le tissu cardiaque par vissage au site d'implantation.

Bien entendu, pour permettre l'échange de données avec un dispositif distant, ces capsules incorporent des moyens émetteurs/récepteurs de communication sans fil avec ce dispositif maître distant

La capsule peut également incorporer un capteur permettant de mesurer localement la valeur d'un paramètre tel que le niveau d'oxygène dans le sang, l'accélération de la paroi cardiaque, etc. Ces capsules peuvent être également des capsules de détection/stimulation composant des moyens pour recueillir des potentiel de dépolarisation du myocarde, et/ou pour appliquer des impulsons de stimulation au site où est implantée la capsule. La capsule porte alors une électrode approprièe, qui peut être notamment constituée par une partie active de la vis d'ancrage La présente invention concerne plus précisément la délivrance des capsules *leadless,* c'est-à-dire leur mise en place au site d'implantation choisi. L'invention n'est pas limitée à un type particulier de capsule, et elle est applicable indifféremment à tout type de capsule *leadless*, quelle que soit sa destination fonctionnelle.

De façon générale, les capsules leadless peuvent être des capsules épicardiques, fixées à la paroi extérieure du coeur, ou bien des capsules en docavitaires, fixées à la paroi intérieure d'une cavité ventriculaire ou auriculaire.

Dans le cas des capsule endocavitaires, les contraintes d'implantation sont accrues du fait de la voie d'approche, qui implique de passer par le réseau veineux périphérique puis de diriger sous amplificateur de brillance la capsule vers le site d'implantation choisi, de façon à la fois précise et parfaitement sécurisée. Ce n'est qu'une fois le site atteint et la capsule ancrée dans la paroi que l'opérateur pourra procéder au "largage" de cette capsule, c'est-à-dire à sa désolidarisation d'avec l'accessoire de pose.

Le US 2009/0204170 A (Cardiac Pacemakers, lnc., décrit une capsule leadless d'électrostimulation ainsi qu'un outil pour sa pose, dans lequel la capsule est amenée par un cathéter jusqu'au site d'implantation à l'intérieur d'un tube porteur appliqué contre la paroi cardiaque, puis progressivement vissée dans cette dernière par un mandrin d'entraînement s'etendant dans la lumière du cathéter.

L'acceptation par les praticiens de la technique des capsules *leadless* endocavitaires repose sur la possibilité de proposer un système de délivrance qui soit capable de sécuriser la pose de ces capsules en répondant à l'ensemble des contraintes suivantes:

-    procédure proche de la pratique courante, qui fasse appel à des gestes bien connus et maîtrisés des praticiens: ponction sous-claviére, insertion et manipulation d'un cathéter via des mandrins préformés pendant la phase d'approche du site d'implantation choisi, fixation de type vis ou barbe, etc. ;
-    environnement standard du bloc opératoire ;
-    limilation des risques de "carottage" des tissus du fait d'un vissage excessif qui pourrait endommager la paroi ou, pire, la perforer (notamment dans le cas de l'implantation dans une paroi mince que le septum interauriculaire) ;
-    possibilité de retrait et/ou de repositionnement post-opératoire en cas de problème, même après largage de la capsule ;
-    limitation des conséquences d'une migration de la capsule en cas de déplacement de celle-ci en phase aigüe pendant l'intervention ;
-    certitude d'une bonne fixation de la capsule avant retrait des accessoires de pose - celle contrainte étant la plus critique de toutes.

L'invention propose à cet effet un ensemble de capsule intracardiaque avec son accessoire d'implantation *in*

*situ* comportant, de manière en elle-même connue notamment d'après le US 2009/0204170 A1 précité : une capsule autonome, comportant un corps tubulaire cylindrique pourvu à l'une de ses extrémités d'une vis d'ancrage hélicoïdale saillante prolongeant axialement le corps cylindrique, et apte à pénétrer dans le tissu de la paroi d'une cavité du coeur, cette capsule comportant au moins un doigt de couplage solidaire du corps cylindrique et faisant saillie radialement vers l'extérieur , et un accessoire d'implantation de la capsule, comportant des moyens dècouplables de support et de guidage de la capsule jusqu'au site d'implantation, et d'entraînement en rotation de cette capsule pour permettre l'entraînement simultané de la vis et son ancrage par pénétration dans la paroi de la cavité du coeur.

De façon caractéristique de l'invention, l'accessoire d'implantation comprend un corps de sonde avec une gaine en matériau déformable portant côté distal un guide hélicoïdal formant lesdits moyens découplables de support, de guidage et d'entraînement en rotation de la capsule. Le guide hélicoïdal prolonge axialement le corps de sonde et est solidaire de celui-ci en rotation et en translation, le diamètre intérieur du guide hélicoïdal est homologue du diamètre extérieur du corps cylindrique de la capsule de façon a pouvoir loger celle-ci à l'intérieur, le(s) doigt(s) de couplage faisant saillie entre les spires du guide hélicoidal, et le sens du guide hélicoidal est inverse de celui de la vis d'ancrage.

La capsule compose de préférence deux doigts de couplage, l'un en partie distale et l'autre en partie proximale,

Dans un premier mode de réalisation, le guide hélicoïdal est une hélice saillante prolongeant axialement l'extrémité distale du corps de sonde, notamment une hélice élastiquement compressible en direction axiale et dont le pas d'hélice est augmenté dans sa partie d'extrémité distale libre. De préférence, la capsule comportant deux doigts de couplage, l'un en partie distale et l'autre en partie proximale, l'écartement en direction axiale de ces deux doigts de couplage est choisi, de manière à procurer une compression de l'hélice lorsque le corps cylindrique de la capsule est complètement logé à l'intérieur du guide hélicoïdal.

Avantageusement, lorsque le corps de la capsule est complètement logé à l'intérieur du guide hélicoidal, cet ensemble comprend un enrobage soluble de protection englobant la capsule munie de sa vis d'ancrage avec le guide hélicoïdal.

La capsule peut comprendre en outre une rampe de réarmement prolongeant le doigt de couplage situé en partie distale, cette rampe formant une fraction de filet hélicoïdal avec un sens d'hèlice inverse de celui du guide hélicoidal, et étant apte à venir en contact sur son flanc proximal avec l'extrémitè libre du guide hélicoïdal.

Dans un deuxième mode de réalisation, l'extrémité distale du corps de sonde porte un tube creux cylindrique s'étendant axialement et formant un logement apte à contenir la capsule, le guide hélicoïdal étant une rainure hélicoïdale formée dans la surface intérieure de ce logement, et il est prevu des moyens pour déployer la vis d'ancrage par un entraînement de type *pin-driven*.

De préférence, le pas du guide hélicoïdal est augmenté dans sa partie d'extrémité distale libre.

De préférence également, la capsule comportant deux doigts de couplage, l'un en partie distale et l'autre en partie proximale, l'écartement en direction axiale de ces deux doigts de couplage est choisi de manière à procurer une compression du ressort lorsque le corps cylindrique de la capsule est complètement logé à l'intérieur du guide hélicoïdal.

De façon gènèrale, l'ensemble peut comprendre en outre un fil souple disposé dans une lumière du corps de sonde et reliant la capsule à l'extrémité proximale du corps de sonde sur toute la longueur de celui-ci, ce fil comprenant au voisinage du point de liaison à la capsule une portion réalisée en un matériau résorbable.

La capsule peut notamment être une capsule de détection/stimulation comportant des moyens de recueil de potentiels de dépolarisation et/ou d'application d'impulsions de stimulation couplés à au moins une électrode portée par la capsule, notamment une électrode constituée par une partie active de la vis d'ancrage, ainsi que des moyens émetteurs/récepteurs de communication sans fil avec un dispositif maître distant.

On va maintenant décrire un exemple de mise en oeuvre de l'invention, en référence aux dessins annexés où les mêmes références numériques désignent d'une figure à l'autre des éléments identiques ou fonctionnellement semblables.

La Figure 1 est une vue perspective montrant la capsule autonome et l'accessoire d'implantation dans une configuration où ces deux éléments sont séparés.

La Figure 2 est homologue de la Figure 1, dans une configuration où la capsule autonome a été accouplée à l'accessoire d'implantation, juste avant la phase d'implantation.

La Figure 3 est homologue de la Figure 2, dans une configuration en cour d'implantation de la capsule dans la paroi de la cavité du coeur, montrant les différentes sollicitations réciproques exercées entre la capsule et l'accessoire d'implantation.

La Figure 4 illustre, en perspective et en coupe, un second mode de rèalisation de l'accessoire d'implantation, avant mise en place de la capsule.

La Figure 5 est homologue de la Figure 4, avec la capsule logée dans l'accessoire d'implantation.

La Figure 6 est une vue agrandie de la partie distale de l'accessoire d'implantation illustré Figure 4.

La Figure 7 illustre une variante perfectionnée mettant en oeuvre un dispositif de type fil d'Ariane.

Les Figures 8a et 8b sont des vues perspectives, selon deux orientations différentes, d'un perfectionnement au mode de réalisation procurant une réversibilité de la pose de la capsule, afin de pouvoir si nécessaire dévisser celle-ci pour la retirer et la réimplanter en un autre site.

On va maintenant décrire un exemple de réalisation de S'invention.

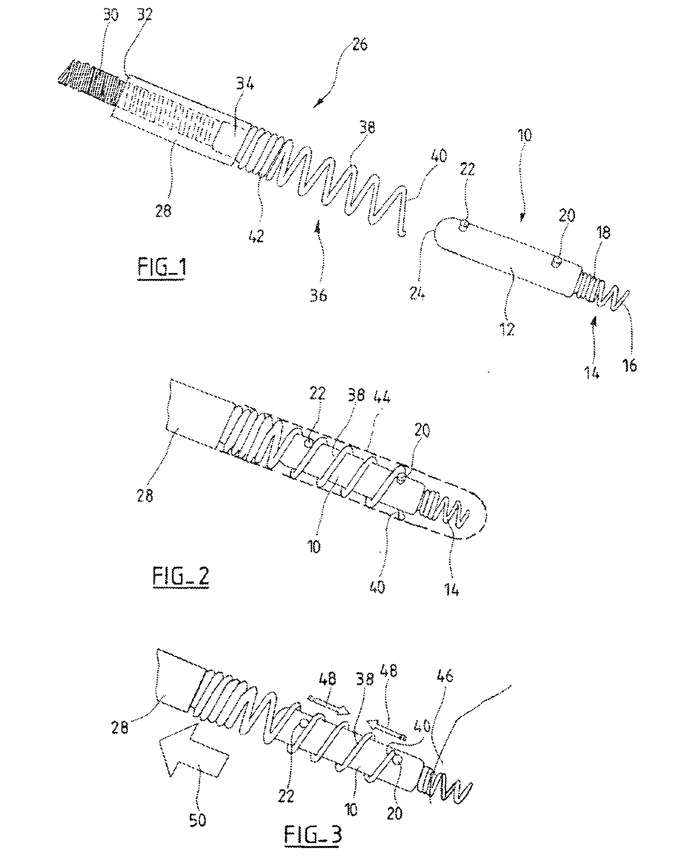

Sur la Figure 1, la référence 10 désigne une capsule autonome de type *leadless*, comprenant de façon en elle-même connue un corps tubulaire 12 cylindrique, pourvu à l'une de ses extrémités d'une vis d'ancrage 14 hélicoïdale saillante prolongeant axialement le corps tubulaire 12 et solidaire de celui-ci en rotation. La vis d'ancrage 14 est avantageusement réalisée avec une partie distale 16 formée sur une longueur de l'ordre de 1,5 à 2 mm de spires non jointives destinées à pénétrer dans le tissu car diaque. Cette partie distale 16 est reliée au corps tubulaire 12 par l'intermédiaire d'une partie de transition mécanique 18 présentant une souplesse en flexion, par exemple une partie formée de spires jointives en l'absence de sollicitation de la vis.

La vis d'ancrage 14 peut être une vis électriquement active, c'est-à-dire jouant, au moins à son extrémité distal, le rôle d'électrode de détection et/ou de stimulation, ou bien une vis passive ne servant qu'à l'ancrage du corps tubulaire 12 dans la paroi de la cavité cardiaque.

Le corps tubulaire 12 inclut divers moyens et circuits d'alimentation, de traitement du signal et de communication sans fil pour permettre l'échange de signaux avec un dispositif maître distant, implanté ou non. Ces aspects sont en eux-mêmes connus et, comme ils ne font pas partie de l'invention, ils ne seront pas dècrits ; on pourra se référer aux diverses publications citées en début de la présente description pour plus de détails à ce sujet.

Le corps tubulaire 12 est cylindrique et, de façon caractéristique de l'invention, porte au moins un doigt de couplage en forme de protubérance axialement savante, dont la fonction sera explicitée plus bas. Dans l'exemple illustré, qui correspond à une configuration avantageuse, la capsule comprend deux de ces doigts de couplage, situés l'un 20 côté distal, l'autre 22 côté proximal, avec un écartement en direction axiale présentant une valeur prédéterminée (on expliquera plus bas l'importance de cette caractéristique). La forme de ces doigts de couplage 20, 22 peut être adaptée de manière à leur donner un profil adouci, atraumatique.

Le corps tubulaire 12 présente une longueur de l'ordre de 5 a 10 mm et un diamètre de l'ordre de 1 à 2 mm (6 French). L'extrémité proximale 24 de la capsule 10 peut être arrondie ou en forme d'ogive pour, d'une part, la rendre atraumatique et, d'autre part, faciliter son couplage avec l'accessoire de pose.

Les Figures 1 à 3 illustrent un premier mode de réalisation de accessoire de pose 26 pour la mise en place, de la capsule 10.

Cet accessoire comprend, essentiellement, un corps 28 structurellement semblable à un corps de sonde de type monopolaire, c'est-à-dire avec une structure en elle-même connue comportant sur toute sa longueur un conducteur spiralé 30 enrobé d'une gaine 32. La gaine 32 est général ment en polyuréthanne, pour limiter les frottements lorsque le corps de sonde est introduit dans un cathéter-guide ou dans le réseau veineux, et pour procurer une meilleure sensibilité et une meilleure transmission du couple de torsion. On notera que, dans le cas de l'invention, le conducteur 30 n'a pas de fonction électrique, il contribue seulement au comportement mécanique du corps de sonde 28 en assurant en outre la radio-opacité de celui-ci.

L'ensemble conducteur 30/gaine 32 procure au corps de sonde 28 une rigidité en torsion suffisante pour pouvoir transmettre un couple de torsion depuis son extrémité proximale jusqu'à son extrémité distale afin d'entraîner en rotation cette extrémité distale. Il est toutefois possible, en variante ou en complément, d'introduire dans la lumière du corps de sonde 28 un mandrin de vissage spécifique, notamment lorsque la gaine 32 ne présente pas une rigidité en torsion sufl°isante pour entraïner directement l'extrémité distale du corps de sonde depuis l'extrémité proximale L'extrémité distale du corps de sonde 28 est pourvue d'un embout 34 auquel est solidarisé un guide hélicoïdal 36 formé, dans ce mode de réalisation, d'une hélice 38 réalisée dans un matériau élastique (par exemple un alliage de type M35N ou rutinol), permettant de rendre l'hélice 38 compressible en direction axiale à la manière d'un ressort de compression hélicoïdal.

De façon caractéristique, cette hélice-ressort 38 présente un pas inversé par rapport au pas de la vis d'ancrage hélicoïdale 16 de la capsule, par exemple un pas à gauche si la vis d'ancrage présente un pas à droite, En outre, l'hélice-ressort 38 présente à son extrémité distale libre un pas légèrement allongé, par exemple sur la spire la plus distale 40

A !'extrémité opposée, l'hélice-ressort 38 est reliée à l'embout 34 par une partie de transition 42 présentant une certaine souplesse en flexion, par exemple une partie 42 formée de spires jointives en l'absence de sollicitation de l'hèlice-ressort 38,

Lors de la fabrication, le corps 12 de la capsule 10 est vissé dans l'hélice-ressort 38, aboutissant ainsi à la configuration illustrée Figure 2. L'entraxe des doigts de couplage 20, 22 est dimensionné de manière à assurer, dans cette configuration, une légère compression de l'hélice-ressort 38 lorsque le doigt de couplage distal 20 amorce la spire 40 qui, comme on l'a exposé plus haut, présente un pas allongé par rapport au reste de l'hélice-ressort 38.

L'ensemble de la partie distale est alors recouvert d'un revêtement soluble 44, par exemple de polyéthyléne glycol (PEG), Cette protection a pour but de protéger la vis d'ancrage 14, l'hélice-ressort 38 et les tissus environnants lors de l'insertion de l'ensemble dans le réseau veineux, Pour limiter le temps de dissolution, il est possible de prévoir pour ce revêtement un profil étagé, évitant une surépaisseur du revêtement autour de la vis d'ancrage 14.

Lors de l'implantation, l'ensemble est introduit jusqu'à la cavité selon une procédure classique, Le corps de sonde 28 étant de construction standard. le praticien retrouvera les sensations classiques de couple. de flexibilité, de glissement, etc.

Une fois la protection PEG 44 complètement dissoute, le médecin positionne l'extrémité de la vis d'ancrage 14 contre la paroi cardiaque, et commence le vissage, par rotation dans le sens horaire (correspondant au pas à droite de la vis d'ancrage 14).

Le couple est transmis depuis l'extrémité proximale du corps de sonde 28 et permet, dans un premier temps, la pénétration de la vis d'ancrage 14 dans le tissu de la paroi 46 de la cavité du coeur. La valeur correspondante du couple permettant cette opération sera désignée $C_{vissage}$

La Figure 3 illustre la configuration de l'ensemble après un vissage complet : la face avant de la capsule 10 vient en butée contre la paroi cardiaque 46 et stoppe ainsi la progression de la vis d'ancrage 14 en générant également en retour une forte augmentation du couple de réaction

Avec une vis d'ancrage d'une sonde standard. si le praticien poursuivait la rotation du corps de sonde et de la vis, le couple augmenterait et dépasserait une limite $C_{carotiage}$. la vis d'ancrage 14 risquant alors de déchirer localement les tissus sous l'effet de la rotation de la vis sans avance de celle-ci, jusqu'à provoquer une lacération des tissus et. a l'extrême, une perforation de la paroi avec risque de tamponnade.

Tel n'est pas le cas avec le dispositif proposé par l'invention : le médecin peut en effet poursuivre sans risque la rotation, toujours dans le sens horaire, du corps de sonde 28 car le couple supplèmentaire apparaissant du fait de la réaction de la vis 14 ancrée dans les tissus est absorbé par la liaison entre l'hélice-ressort 38 et la capsule 10. Plus précisèment. l'élasticité en compression de l'hélice-ressort 38 est choisie de manière à dèfinir un couple de glissement $C_{gilssement}$ inférieur à la limite $C_{carottage}$ du carottage, de sorte que lorsque le couple $C_{glissement}$ est atteint, la poursuite de la rotation du corps de sonde 28 dans le sens horaire amorce la rotation de autour de la capsule 10, grâce au pas inversé de l'hèlice-ressort 38, Cette dernière se dégage alors progressivement de la capsule 10 par dévissage (du fait de l'inversion du sens du pas). On notera que la dimension de pas légèrement accrue de la dernière spire 40 permet de générer une compression des spires de l'hélice-ressort 38 entre les deux doigts de couplage 20 et 22 (flèches 48) et donc une force d'appui croissante sur ces doigts de couplage, ceci jusqu'à la libération du doigt distal 20, situation qui définit un couple de débrayage $C_{débrayage}$ permettant alors le découplage de la capsule 10 et du corps de sonde 28 (flèche 50).

La géométrie des différents éléments qui assurent cette interaction. ainsi que l'élasticité en compression de l'hélice-ressort, sont choisis de manière à vérifier la relation:

$$C_{vissage} < C_{glissement} < C_{débrayage} < C_{carottage}$$

$C_{vissage}$ désignant le couple de vissage dans les tissus.
$C_{glissement}$ désignant le couple supplémentaire absorbé par la liaison entre l'hélice et la capsule,
$C_{debravage}$ dèsignant le couple atteint à la libération du doigt distal , et
$C_{carottage}$ désignant le couple limite au-delà duquel la rotation de la vis d'ancrage risque provoquer un déchirement des tissus de la paroi

On notera que le système de dèbrayage, situé à proximité de la vis d'ancrage 14 et donc en partie d'extrémité distale de l'ensemble, n'est pas dépendant du comportement en torsion du corps de sonde 28, à la différence par exemple d'un système de limitation de couple qui serait placé côté proximal.

D'autre part, on notera que lors du débrayage la longueur comprimée de l'hélice-ressort 36 est maximale, ce qui permet de garantir une reproductibilité maximale du couple de débrayage.

Le mécanisme que l'on vient de décrire permet d'absorber la montée progressive du couple du fait de la réaction de la vis d'ancrage 14 une fois celle-ci complètement introduite dans la paroi de la cavité cardiaque, avec un double bénéfice :

certitude du vissage complet de la capsule 10, et
suppression de tout risque de tamponnade.

L'ensemble de l'opération est transparente pour le médecin, dans la mesure où un simple et unique mouvement de rotation horaire depuis l'extrémité proximale du corps de sonde 28 permet d'assurer à la fois la fixation complète de la capsule et son largage.

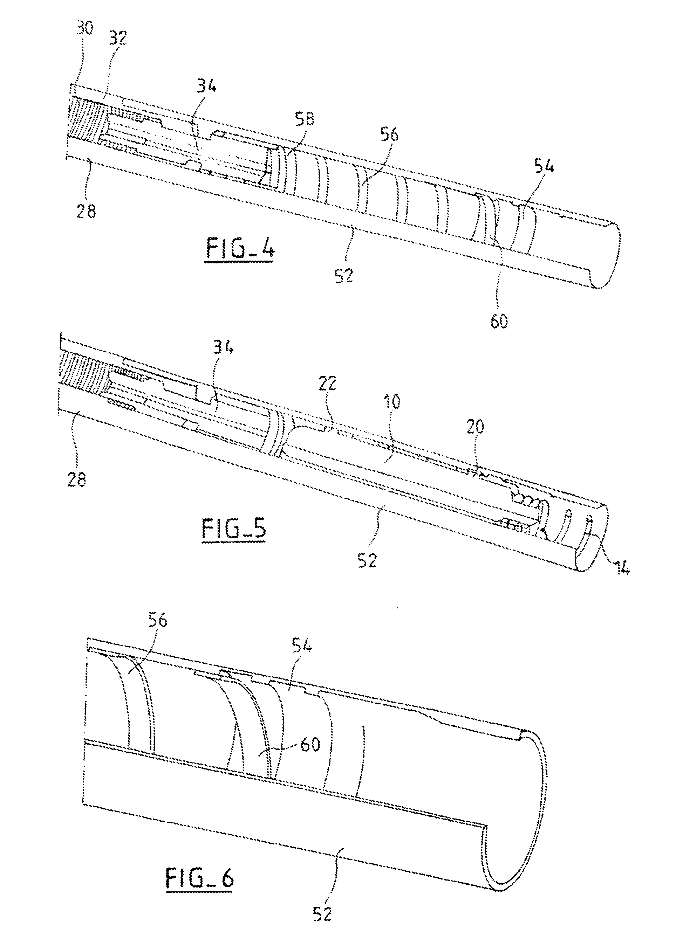

On va maintenant décrire en référence aux Figures 4 à 6 un second mode de réalisation de l'invention,

En particulier, ce second mode de réalisation est bien adapté aux cas où le corps de sonde 28 est un système standard de type dit *pin driven*, où le praticien maintient d'une main l'extrémité proximale du corps de sonde et de l'autre main fait tourner, directement ou par l'intermédiaire d'un outil, la fiche (*pin*) dépassant de cette extrémité proximale. Concrètement. la fiche est solidaire du conducteur axial 30 s'étendant à l'intérieur du corps de sonde ce conducteur étant alors libre en rotation par rapport à la gaine 32 et étant relié à son extrémité distale à l'embout 34.

Ce second mode de réalisation permet par ailleurs d'évier le recours d'un revêtement en PEG soluble pour la protection de la vis, dans la mesure où cette dernière pourra être rètractée à intérieur d'un tube protecteur pendant toute la durée de la descente intraveineuse.

Plus précisément, le corps de sonde 28 porte à son extrémité un tube creux cylindrique 52, Ce tube présente un diamètre intérieur correspondant au diamètre extérieur de la capsule 10 (cf Figure 5) et une longueur permettant de loger celle-ci entièrement, y compris la vis d'ancrage 14, à l'intérieur du tube creux.

Le tube creux 52 est muni d'une rainure hélicoïdale 54 (visible notamment sur la vue agrandie de la Figure 6) formée dans la surface intérieure du tube débouchant dans un évidement interne circulaire. Le doigt de couplage 20 coulisse dans cette rainure hélicoïdale lorsque la broche du connecteur est actionnée (technique pin *driven),* entrainant ainsi la vis 14 hors du tube creux 52.

Le guide hélicoïdal formant ressort est représenté en 56. Il se présente sous forme d'un ruban plat 56 en matériau élastiquement déformable en compression, avec une extrémité proximale 58 solidaire de l'embout 34 et une extrémité distale hbre 60 présente sur sa dernière spire un pas de dimension légèrement accrue, de la même façon que dans le mode de réalisation précédent.

Le sens du pas de la rainure hélicoïdale 54 est le même que celui de la vis d'ancrage 14 (pas à droite), en revanche le pas du ressort plat 56 formant guide héhcoïdal est un pas inversé (pas à gauche).

Avec cette configuration, la rotation de la broche du connecteur côté proximal du corps de sonde 28 entraînera une rotation de l'embout 34 et simultanément de l'ensemble constitué par la capsule 10 et le ressort 56 dans un mouvement hélicoïdal vers l'avant par rapport au tube 52, entraînant ainsi progressivement le déploiement de la vis 14 hors du tube 52, puis le vissage de celle-ci dans la paroi de la cavité du coeur, jusqu'à ce que la face avant de la capsule vienne en appui contre cette paroi.

La poursuite du vissage provoque, par l'action du ressort hélicoïdal 56 sur les doigts de couplage 20 et 22, la séparation de la capsule 10 d'avec le ressort 56, permettant le largage progressif de cette dernière avec la fonction de débrayage décrite plus haut à propos du premier mode de réalisation, qui empêche tout endommagement des tissus par la vis d'ancrage. La cinématique et les contraintes Sur les valeurs de couple exposées en détail à propos du premier mode de réalisation sont applicables de la même façon à ce second mode de rèalisation.

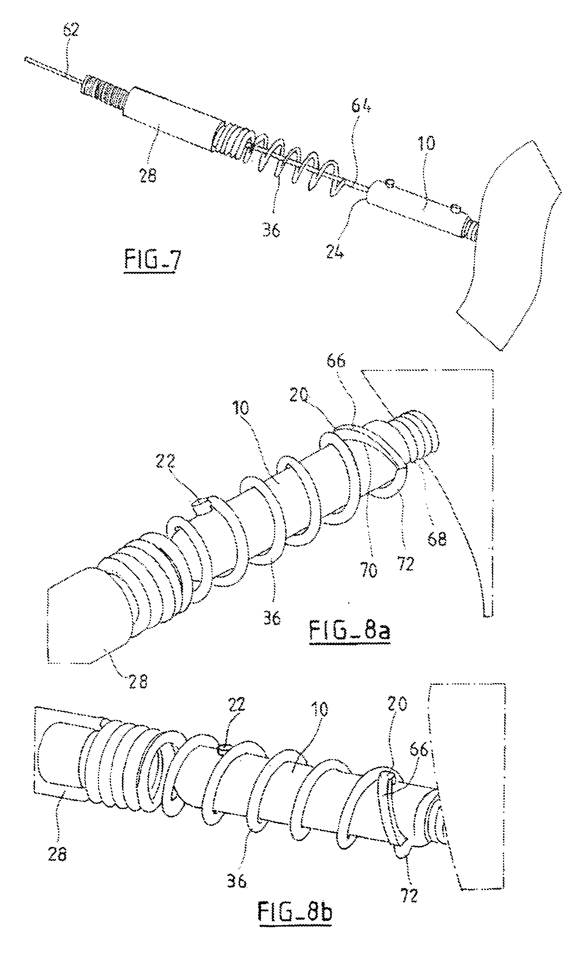

La Figure 7 illustre un perfectionnement avantageux qui peut être mis en oeuvre avec l'un ou l'autre des deux modes de réalisation précédemment décrits. et qui est conçu pour permettre un repositionnement à court ou moyen terme de la capsule 10 après son largage.

Dans ce perfectionnement la liaison entre le corps de sonde 28 et la capsule 10, qui sont des éléments dissociables, est doublée par un "fil d'Ariane" 62 relié à la capsule 10 du côté distal et dépassant du corps de sonde du côté proximal (non représenté).

Une fois la capsule 10 implantée, le bon fonctionnement de celle-ci est testé, notamment le bon établissement de la communication sans fil enture la capsule et le dispositif maître distant. Lorsque la capsule est correctement fixée, le corps de sonde 28 est complètement retiré, et un surplus de longueur du fil d'Ariane 62 est laissé de manière a dépasser en dehors du patient, sous le pansement.

A ce stade, le fil d'Ariane permet de récupérer la capsule en cas de déplacement en phase aigüe, par simple traction sur le fil,

Le fil d'Ariane porte une région 64 en matériau résorbable à l'endroit de sa raison avec la capsule 10. par exemple sur une longueur de 3 à 5 mm, ce qui permet le retrait définitif du fil d'Ariane par une simple traction, par exemple un mois après l'intervention.

Tout ou partie du fil d'Ariane 62 peut contenir un agent actif de type DSP ou un traitement de surface permettant de stopper toute propagation d'infection entre la partie émergeante (sous le pansement) et la partie introduite dans le réseau veineux.

En cas de test négatif juste après l'implantation ou en cas de dysfonctionnement ultérieur, il est possible de réengager l'hèlice-ressort 36 sur la capsule grâce au guidage du fil d'Ariane 62 et à la forme arrondie de la partie arrière 24 de la capsule 10. Cette dernière peut alors être dévissée de la paroi par une rotation du corps de sonde 28 dans le sens antihoraire, puis replacée sur un autre site par le même principe que ce qui a été décrit plus haut, par une rotation dans le sens horaire.

Le fil d'Ariane peut être coloré de couleurs différentes pour chacune des capsules implantées, de manière à identifier plus tapement la capsule concerné (auriculaire, ventrlculaire, ..) dans l'éventualité d'une réintervention.

La technique de l'invention procure donc une double sécurité lors du largage de la capsule : la première sécurité résulte du système de débrayage permettant d'épier tout carottage de la paroi cardiaque, le second garantit au praticien la possibilitè, même après largage, de récupérer à moyen terme la capsule en cas de difficulté au moyen ou fil d'Ariane. Les Figures 8a et 8b illustrent selon deux orientations différentes, un perfectionnement procurant une réversibilité de la pose de la capsule, afin de pouvoir si nécessaire accoupler à nouveau le corps de sonde 28 à la capsule 10 de manière à dévisser celle-ci pour la retirer et la réimplanter éventuellement en un autre site,

Au moment de la remise en place du corps de sonde sur la capsule, le réarmement *in situ* de l'hélice-ressort 36 nécessite d'aménager sur la capsule 10 dans la région distale une rampe de compression 66 prolongeant le doigt de couplage distal 20. Cette rampe présente, comme illustré sur les Figures Sa et 8b, une forme hélicoïdale 68 s'étendant sur la longueur d'une fraction de spire, avec un pas à droite (inverse de celui de l'hélice-ressort 36) et présentant un flanc proximal 70 contre lequel viendra glisser J'extrémité libre 72 de l'hélice-ressort 36. La rampe de compression 66 est rendue nécessaire par le fait que, en l'absence de cette rampe, l'hélice-ressort 36 non compressée au moment de l'accostage avec la capsule 10 puis du vissage sur le doigt de couplage proximal 22, viendrait engager le doigt de couplage distal 20 par son côté distal, de sorte que le mécanisme de débrayage décrit plus haut ne fontionnerait plus.

## Revendications

**1.** Un ensemble de capsule intracardiaque (10) avec son accessoire (26) d'implantation *in situ,* comprenant :

une capsule autonome (10), comportant un corps tubulaire (12) cylindrique pourvu à l'une de ses extrémités d'une vis d'ancrage (14) hélicoïdale saillante prolongeant axialement le corps cylindrique, et apte à pénétrer dans le tissu de la paroi (46) d'une cavité du coeur, cette capsule (10) comportant au moins un doigt de couplage (20, 22) solidaire du corps cylindrique et faisant saillie radialement vers l'extérieur ; et
- un accessoire (26) d'implantation de la capsule, comportant des moyens découplables de support et de guidage de la capsule jusqu'au site d'implantation, et d'entraînement en rotation de cette capsule pour permettre l'entraînement simultané de la vis et son ancrage par pénétration dans la paroi de la cavité du coeur,

ensemble **caractérisé en ce que** :

- l'accessoire d'implantation (26) comprend un corps de sonde (23) avec une gaine (30, 32) en matériau déformable portant côté distal un guide hélicoïdal (36 ; 52) formant lesdits moyens découplables de support, de guidage et d'entraînement en rotation de la capsule, où :

• le guide hélicoïdal prolonge axialement le corps, de sonde et est solidaire de celui-ci en rotation et en translation :
• le diamètre intérieur du guide hélicoidal est homologue du diamètre extérieur du corps cylindrique de la capsule de façon à pouvoir loger celle-ci à l'intérieur, ie(s) doigt(s) de couplage faisant saillie entre les spires du guide hélicoïdal ; et
• le sens d'hélice du guide hélicoïdal (36) est inverse de celui de la vis d'ancrage (14).

**2.** L'ensemble de la revendication 1, dans lequel la capsule comporte deux doigts de couplage, l'un (20) en partie distale et l'autre (22) en partie proximale.

**3.** L'ensemble de la revendication 1, dans lequel le guide hélicoïdal est une hélice saillante (38) prolongeant axialement l'extrémité distale du corps de sonde.

**4.** L'ensemble de la revendication 3, dans lequel l'hélice (38) est élastquement compressible en direction axiale.

**5.** L'ensemble de la revendication 4, dans lequel le pas de l'hélice (38) est augmenté dans sa partie d'extrémité distale libre (40).

**6.** L'ensemble de la revendication 5, dans lequel la capsule comporte deux doigts de couplage, l'un (20) en partie distale et l'autre (22) en partie proximale, et dans lequel l'écartement en direction axiale de ces deux doigts de couplage est choisi de manière à procurer une compression de l'hélice (38) lorsque le corps cylindrique de la capsule est complètement logé à l'intérieur du guide hélicoïdal.

**7.** L'ensemble de la revendication 3, dans lequel, lorsque le corps cylindrique de la capsule est complètement logé à l'intérieur du guide hélicoïdal; cet ensemble comprend un enrobage soluble de protection (44) englobant la capsule munie de sa vis d'ancrage avec le guide hélicoïdal.

8. L'ensemble de la revendication 3, dans lequel la capsule comprend en outre une rampe de réarmement (66) prolongeant le doigt de couplage (20) situé en partie distale, cette rampe formant une fraction de filet hélicoïdal avec un sens d'hélice inverse de celui du guide hélicoïdal, et étant apte à venir en contact sur son flanc proximal (70) avec l'extrémité libre (72) du guide hélicoïdal.

9. L'ensemble de la revendication 1, dans lequel l'extrémité distale du corps de sonde porte un tube creux cylindrique (52) s'étendant axialement et fermant un logement apte à contenir la capsule et le guide hélicoïdal, et il est prévu des moyens pour déployer la vis d'ancrage par un entraînement de type *pin-driven*.

10. L'ensemble de la revendication 9, dans lequel le pas du guide hélicoïdal est augmenté dans sa partie d'extrémité distale libre (60).

11. L'ensemble de la revendication 10, dans lequel dans lequel la capsule comporte deux doigts de couplage, l'un (20) en partie distale et l'autre (22) en partie proximale, et dans lequel l'écartement en direction axiale de ces deux doigts de couplage est choisi de manière à procurer une compression du ressort (56) lorsque le corps cylindrique de la capsule est complètement logé à l'intérieur du guide hélicoïdal.

12. l'ensemble de la revendication 1, comprenant en outre un fil souple (62) disposé dans une lumière du corps de sonde et reliant la capsule à l'extrémité proximale du corps de sonde sur toute la longueur de celui-ci, ce fil comprenant au voisinage du point de liaison à la capsule une portion (64) réalisée en un matériau résorbable,

13. L'ensemble de la revendication 1, dans lequel la capsulle (10) est une capsule de détection/stimulation comportant des moyens de recueil de potentiels de dépolarisation et/ou d'application d'impulsions de stimulation couples a au moins une électrode portée par la capsule, notamment une électrode constituée par une partie active de la vis d'ancrage, ainsi que des moyens émetteurs/récepteurs de communication sans fil avec un dispositif maître distant.

14. Une capsule intracardiaque autonome (10) pour un ensemble selon l'une des revendications 1 à 13, cette capsule comportant un corps tubulaire (12) cylindrique pourvu à l'une de ses extrémités d'une vis d'ancrage (14) hélicoïdale saillante prolongeant axialement le corps cylindrique, et apte à pénétrer dans le tissu de la paroi (46) d'une cavité du coeur,
**caractérisée en ce qu'**elle comporte au moins un doigt de couplage (20, 22) solidaire du corps cylindrique et faisant saillie radialement vers l'extérieur.

15. Un accessoire d'implantation in situ (26) pour un ensemble selon l'une des revendications 1 à 13, cet accessoire (26) comportant des moyens découplables de support et de guidage d'une capsule jusqu'au site d'implantation, et d'entraînement en rotation de cette capsule pour permettre l'entraînement simultané de la vis et son ancrage par pénétration dans la paroi de la cavité du coeur,
**caractérisé en ce qu'**il comprend un corps de sonde (28) avec une gaine (30, 32) en matériau déformable portant côté distal un guide hélicoïdal (36 ; 52) formant lesdits moyens découplables de support, de guidage et d'entraînement en rotation de la capsule, où :

- le guide hélicoïdal prolonge axialement le corps de sonde et est solidaire de celui-ci en rotation et en translation ;
- le diamètre intérieur du guide hélicoïdal est homologue du diamètre extérieur du corps cylindrique de la capsule de façon à pouvoir loger celle-ci à l'intérieur, le(s) doigt(s) de couplage faisant saillie entre les spires du guide hélicoïdal ; et
- le sens d'hélice du guide hélicoïdal (36) est inverse de celui de la vis d'ancrage (14).

## Claims

1. Assembly of intracardiac capsule (10) with its accessory (26) for implantation *in situ*, comprising:

- an autonomous capsule (10), having a cylindrical tubular body (12) provided at one of its ends with a projecting helical anchoring screw (14) that forms an axial continuation of the cylindrical body and that is able to penetrate the tissue of the wall (46) of a cavity of the heart, this capsule (10) having at least one coupling finger (20, 22) integral with the cylindrical body and projecting radially outwards; and

- an accessory (26) for implantation of the capsule, having disconnectable means for supporting and guiding the capsule to the implantation site, and for driving this capsule in rotation in order to permit simultaneous driving of the screw and the anchoring thereof by penetration in the wall of the cavity of the heart,

said assembly being **characterized in that**:

- the implantation accessory (26) comprises a lead body (28) with a sheath (30, 32) of deformable material which, at the distal end, carries a helical guide (36; 52) forming said disconnectable means for supporting and guiding the capsule and for driving it in rotation, where:

  • the helical guide forms an axial continuation of the lead body and is integral therewith in rotation and in translation;
  • the internal diameter of the helical guide is homologous to the external diameter of the cylindrical body of the capsule so as to be able to house the capsule inside the helical guide, with the coupling finger(s) projecting between the turns of the helical guide; and
  • the helix direction of the helical guide (36) is the opposite to that of the anchoring screw (14).

2. Assembly according to Claim 1, in which the capsule has two coupling fingers, one (20) in the distal part and the other (22) in the proximal part.

3. Assembly according to Claim 1, in which the helical guide is a projecting helix (38) forming an axial continuation of the distal end of the lead body.

4. Assembly according to Claim 3, in which the helix (38) is elastically compressible in the axial direction.

5. Assembly according to Claim 4, in which the pitch of the helix (38) is increased in its free distal end part (40).

6. Assembly according to Claim 5, in which the capsule has two coupling fingers, one (20) in the distal part and the other (22) in the proximal part, and in which the axial distance between these two coupling fingers is chosen in such a way as to provide a compression of the helix (38) when the cylindrical body of the capsule is completely housed inside the helical guide.

7. Assembly according to Claim 3, in which, when the cylindrical body of the capsule is completely housed inside the helical guide, this assembly comprises a soluble protective coating (44) that surrounds the capsule, with its anchoring screw, and the helical guide.

8. Assembly according to Claim 3, in which the capsule further comprises a rearming ramp (66) forming a continuation of the coupling finger (20) situated in the distal part, this ramp forming a helical thread portion with a helix direction opposite to that of the helical guide and being able to come into contact, at its proximal flank (70), with the free end (72) of the helical guide.

9. Assembly according to Claim 1, in which the distal end of the lead body carries a hollow cylindrical tube (52) extending axially and forming a housing for containing the capsule and the helical guide, and means are provided for deploying the anchoring screw using a drive mechanism of the pin-driven type.

10. Assembly according to Claim 9, in which the pitch of the helical guide is increased in its free distal end part (60).

11. Assembly according to Claim 10, in which the capsule has two coupling fingers, one (20) in the distal part and the other (22) in the proximal part, and in which the axial distance between these two coupling fingers is chosen in such a way as to provide a compression of the spring (56) when the cylindrical body of the capsule is completely housed inside the helical guide.

12. Assembly according to Claim 1, further comprising a flexible wire (62) disposed in a lumen of the lead body and connecting the capsule to the proximal end of the lead body along the entire length thereof, this wire comprising, near the point of attachment to the capsule, a portion (64) made of a resorbable material.

13. Assembly according to Claim 1, in which the capsule (10) is a detection/stimulation capsule with means for receiving depolarization potentials and/or applying stimulation pulses coupled to at least one electrode carried by the capsule, in particular an electrode formed by an active part of the anchoring screw, and also transceiver means for wireless communication with a remote master device.

14. Autonomous intracardiac capsule (10) for an assembly according to one of Claims 1 to 13, this capsule having a cylindrical tubular body (12) provided at one of its ends with a projecting helical anchoring screw (14) that forms an axial continuation of the cylindrical body and that is able to penetrate the tissue of the wall (46) of a cavity of the heart, **characterized in that** it has at least one coupling finger (20, 22) integral with the cylindrical body and projecting radially outward.

15. Accessory (26) for *in situ* implantation for an assembly according to one of Claims 1 to 13, this accessory (26) having disconnectable means for supporting and guiding a capsule to the implantation site, and for driving this capsule in rotation in order to permit simultaneous driving of the screw and the anchoring thereof by penetration in the wall of the cavity of the heart, **characterized in that** it comprises a lead body (28) with a sheath (30, 32) of deformable material which, at the distal end, carries a helical guide (36; 52) forming said disconnectable means for supporting and guiding the capsule and driving it in rotation, where:

   - the helical guide forms an axial continuation of the lead body and is integral therewith in rotation and in translation;
   - the internal diameter of the helical guide is homologous to the external diameter of the cylindrical body of the capsule so as to be able to house the capsule inside the helical guide, with the coupling finger(s) projecting between the turns of the helical guide; and
   - the helix direction of the helical guide (36) is the opposite to that of the anchoring screw (14).

**Patentansprüche**

1. Einheit einer intrakardialen Kapsel (10) mit ihrem Zubehör (26) zur in-situ-Implantation, die enthält:

   - eine autonome Kapsel (10), die einen zylindrischen rohrförmigen Körper (12) aufweist, der an einem seiner Enden mit einem vorstehenden Spiralankerschraube (14) versehen ist, die den zylindrischen Körper axial verlängert und in das Gewebe der Wand (46) einer Höhle des Herzens eindringen kann, wobei diese Kapsel (10) mindestens einen Kopplungsfinger (20, 22) aufweist, der fest mit dem zylindrischen Körper verbunden ist und radial nach außen vorsteht; und
   - ein Implantationszubehör (26) der Kapsel, das entkoppelbare Einrichtungen zum Tragen und Führen der Kapsel bis zur Implantationsstelle und zum Drehantrieb dieser Kapsel aufweist, um den gleichzeitigen Antrieb der Schraube und ihre Verankerung durch Eindringen in die Wand der Höhle des Herzens zu erlauben,

   Einheit, die **dadurch gekennzeichnet ist, dass**:

   - das Implantationszubehör (26) einen Sondenkörper (28) mit einer Hülle (30, 32) aus verformbarem Material enthält, der am distalen Ende eine spiralförmige Führung (36; 52) trägt, die die entkoppelbaren Trag-, Führungs- und Drehantriebseinrichtungen der Kapsel formt, wobei:

      • die spiralförmige Führung den Sondenkörper axial verlängert und mit diesem in Drehung und Translation fest verbunden ist;
      • der Innendurchmesser der spiralförmigen Führung dem Außendurchmesser des zylindrischen Körpers der Kapsel entspricht, um diese im Inneren aufzunehmen, wobei der (die) Koppelfinger zwischen den Windungen der spiralförmigen Führung vorsteht (vorstehen); und
      • die Schraubenrichtung der spiralförmigen Führung (36) entgegengesetzt zu derjenigen der Ankerschraube (14) ist.

2. Einheit nach Anspruch 1, bei der die Kapsel zwei Koppelfinger aufweist, den einen (20) am distalen Teil und den anderen (22) am proximalen Teil.

3. Einheit nach Anspruch 1, bei der die spiralförmige Führung eine vorstehende Schraubenlinie (38) ist, die das distale Ende des Sondenkörpers axial verlängert.

4. Einheit nach Anspruch 3, bei der die Schraubenlinie (38) in axialer Richtung elastisch komprimierbar ist.

5. Einheit nach Anspruch 4, bei der die Steigung der Schraubenlinie (38) an ihrem freien distalen Endteil (40) erhöht ist.

6. Einheit nach Anspruch 5, bei der die Kapsel zwei Koppelfinger aufweist, den einen (20) im distalen Teil und den anderen (22) im proximalen Teil, und bei der die Entfernung dieser zwei Koppelfinger in axialer Richtung so gewählt wird, dass eine Komprimierung der Schraubenlinie (38) hervorgerufen wird, wenn der zylindrische Körper der Kapsel vollständig im Inneren der spiralförmigen Führung untergebracht ist.

7. Einheit nach Anspruch 3, bei der, wenn der zylindrische Körper der Kapsel vollständig im Inneren der spiralförmigen Führung untergebracht ist, diese Einheit eine lösliche Schutzumhüllung (44) enthält, die die mit ihrer Ankerschraube mit der spiralförmigen Führung versehene Kapsel umfasst.

8. Einheit nach Anspruch 3, bei der die Kapsel außerdem eine Wiederspannrampe (66) enthält, die den im distalen Teil befindlichen Koppelfinger (20) verlängert, wobei diese Rampe einen Gewindegangteil mit einer umgekehrten Schraubenlinienrichtung zu derjenigen der spiralförmigen Führung formt, und in der Lage ist, auf ihrer proximalen Flanke (70) mit dem freien Ende (72) der spiralförmigen Führung in Kontakt zu kommen.

9. Einheit nach Anspruch 1, bei der das distale Ende des Sondenkörpers ein zylindrisches Hohlrohr (52) trägt, das sich axial erstreckt und eine Aufnahme formt, die die Kapsel und die spiralförmige Führung enthalten kann, und Einrichtungen vorgesehen sind, um die Ankerschraube durch einen Antrieb der Art pin-driven zu spreizen.

10. Einheit nach Anspruch 9, bei der die Steigung der spiralförmigen Führung in ihrem freien distalen Endbereich (60) erhöht wird.

11. Einheit nach Anspruch 10, bei der die Kapsel zwei Koppelfinger aufweist, einen (20) im distalen Teil und den anderen (22) im proximalen Teil, und bei der die Entfernung dieser zwei Koppelfinger in axialer Richtung so gewählt wird, dass eine Komprimierung der Feder (56) hervorgerufen wird, wenn der zylindrische Körper der Kapsel vollständig im Inneren der spiralförmigen Führung aufgenommen ist.

12. Einheit nach Anspruch 1, die weiter einen geschmeidigen Draht (62) enthält, der in einem Langloch des Sondenkörpers angeordnet ist und die Kapsel mit dem proximalen Ende des Sondenkörpers über dessen ganze Länge verbindet, wobei dieser Draht in der Nähe des Verbindungspunkts mit der Kapsel einen Abschnitt (64) enthält, der aus einem resorbierbaren Material hergestellt ist.

13. Einheit nach Anspruch 1, bei der die Kapsel (10) eine Erfassungs-/Stimulationskapsel ist, die Einrichtungen zur Sammlung von Depolarisations-Potentialen und/oder zum Anlegen von Stimulationsimpulsen, die mit mindestens einer von der Kapsel getragenen Elektrode gekoppelt sind, insbesondere einer Elektrode, die aus einem aktiven Teil der Ankerschraube besteht, sowie Sende-/Empfangseinrichtungen zur drahtlosen Verbindung mit einer fernen Hauptvorrichtung aufweist.

14. Autonome intrakardiale Kapsel (10) für eine Einheit nach einem der Ansprüche 1 bis 13, wobei diese Kapsel einen zylindrischen rohrförmigen Körper (12) aufweist, der an einem seiner Enden mit einer vorstehenden Spiralankerschraube (14) versehen ist, die den zylindrischen Körper axial verlängert und in das Gewebe der Wand (46) einer Höhle des Herzens eindringen kann, **dadurch gekennzeichnet, dass** sie mindestens einen Koppelfinger (20, 22) aufweist, der fest mit dem zylindrischen Körper verbunden ist und radial nach außen vorsteht.

15. Zubehör zur in-situ-Implantation (26) für eine Einheit nach einem der Ansprüche 1 bis 13, wobei dieses Zubehör (26) entkoppelbare Trag- und Führungseinrichtungen einer Kapsel bis zur Implantantionsstelle und zum Drehantrieb dieser Kapsel aufweist, um den gleichzeitigen Antrieb der Schraube und ihre Verankerung durch Eindringen in die Wand der Höhle des Herzens zu erlauben, **dadurch gekennzeichnet, dass** es einen Sondenkörper (28) mit einer Hülle (30, 32) aus verformbarem Material enthält, der auf der distalen Seite eine spiralförmige Führung (36; 52) trägt, die die entkoppelbaren Einrichtungen zum Tragen, Führen und Drehantrieb der Kapsel formt, wobei:

- die spiralförmige Führung den Sondenkörper axial verlängert und in Drehung und Translation fest mit diesem verbunden ist;

- der Innendurchmesser der spiralförmigen Führung dem Außendurchmesser des zylindrischen Körpers der Kapsel entspricht, um diese im Inneren aufzunehmen, wobei der (die) Kopplungsfinger zwischen den Windungen der spiralförmigen Führung vorsteht (vorstehen); und

- die Schraubenrichtung der spiralförmigen Führung (36) umgekehrt zu derjenigen der Ankerschraube (14) ist.

FIG_1

FIG_2

FIG_3

FIG_4

FIG_5

FIG_6

EP 2 394 695 B1

FIG_7

FIG_8a

FIG_8b

**EP 2 394 695 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20070088397 A1
- WO 2007047681 A2
- US 20080136004 A1
- US 20090204170 A
- US 20090204170 A1